# EUROPEAN PATENT APPLICATION

(11) **EP 1 698 336 A1**
(43) Date of publication of application: **06.09.2006**
(21) Application number: 05101540.2
(22) Date of filing: 01.03.2005
(51) Int. Cl.: A61K 31/4178, A61K 31/57, A61P 31/10, A61K 31/4725

(54) **Antifungal compositions comprising Sertaconazole and either Hydrocortisone or an antibacterial agent**

(71) Applicant: FERRER INTERNACIONAL, S.A., 08028 Barcelona (ES)
(72) Inventor: Palacin, Celia, 08017 Barcelona (ES); Guerrero, Marta, 08024 Barcelona (ES); Torres, Jesus, 08022 Barcelona (ES); Raga, Manuel, 08024 Barcelona (ES); Guglietta, Antonio, 08750 Molins de Rei (ES)
(74) Representative: Barlocci, Anna

(57) **Abstract**

Pharmaceutical compositions useful for treating dermal diseases caused by fungi and yeasts with inflammation and/or associated with a bacterial infection. Such compositions contain a therapeutically effective amount of Sertaconazole, or a pharmaceutically acceptable salt, solvate, isomer thereof or mixtures thereof; and a therapeutically effective amount of at least one compound selected from the group consisting of Hydrocortisone, a pharmaceutically acceptable ester of Hydrocortisone, an antibacterial agent or mixtures thereof.

## Description

The present invention relates to topical formulations useful for treating dermal diseases, caused by fungi and yeasts with inflammation and/or associated with a bacterial infection. In particular the present invention relates to stable topical formulations containing an antifungal agent and an anti-inflammatory steroid. More particularly, the present invention relates to topical formulations containing Sertaconazole and Hydrocortisone or an antibacterial agent, or a mixture thereof.

### Background of the invention

Sertaconazole, INN of 1-[2-[(7-Chlorobenzo[b]thien-3-yl)methoxy]-2-(2,4-dichlorophenyl)ethyl]-1 H-imidazole and CAS REG No. 99592-32-2 is a useful antifungal agent for treatment of diseases caused by fungi and yeasts in man and in animals. Sertaconazole, as well as its pharmaceutically acceptable addition salts, is disclosed in EP 151.477. The R-(-)-enantiomer of Sertaconazole (R-(-)-Sertaconazole) is disclosed in PCT application WO 03/68770 as well as its pharmaceutically acceptable addition salts.

The antibacterial agent 1-cyclopropyl-8-methyl-7-[5-methyl-6-(methylamino)-3-pyridinyl]-4-oxo-1,4-dihydro-3-quinolinecarboxylic acid (GF-001001-00) is described in US 6.335.447. This compound is a non-fluorinated quinolone with a wide range of activity against bacteria.

A variety of methods have been used for the treatment of fungal infections including the use of potassium iodide, Whitfield's ointment, undecylenic acid, antibiotics (e.g. Nystatin and Amphotericin B), Griseofulvin and imidazole antifungal agents such as Bifonazole, Butoconazole, Clotrimazole, Econazole, Fenticonazole, Flutrimazole, lsoconazole, Ketoconazole, Miconazole, Omoconazole, Oxiconazole, Parconazole, Sertaconazole, Sulconazole and Tioconazole.

Imidazole antifungal agents are the first broad-spectrum antifungal agents and are of considerable importance in clinical practice. Their broad spectrum of antifungal activity against most pathogenic fungi, has provided an important advance in antifungal therapy.

A large number of suitable imidazoles have been described in the literature and are well-known to those skilled in the art. Examples of suitable imidazole antifungal agents include Bifonazole, Butoconazole, Clotrimazole, Econazole, Fenticonazole, Flutrimazole, lsoconazole, Ketoconazole, Miconazole, Omoconazole, Oxiconazole, Parconazole, Sertaconazole, Sulconazole and Tioconazole.

The infections caused by fungi and yeasts are commonly associated with signs of erythema and scaling and with symptoms of itching or painful burning. Clinical treatment for fungal disease requires at least two to four weeks for complete relief of symptoms. It has been found that fungal infections can be effectively treated with a combination of corticosteroids and imidazole antifungal agents. It is known that the sensitivity of fungal organisms varies with their life cycles; spores are more resistant to treatment than are mycelia. Steroids may induce fungal spores to produce mycelia, thereby making them more sensitive to treatment. Also, steroids are known to produce vasoconstriction at the site of application. This activity may delay or prevent the elimination of the antifungal agent from the application site, permitting the antifungal agent to remain in the epidermis for longer periods of time. It is therefore believed that a locally applied anti-inflammatory agent would offer direct and immediate relief for the inflammatory component of the lesion. The combination product should then provide fast relief of symptoms and eradicate the infection. Based on this concept, certain combinations of an antifungal agent and an anti-inflammatory agent have been developed for treatment of fungal diseases.

However, the antifungal and corticoid combinations are not devoid of side effects. Reported side effects for a commercially available combination, Lotricomb cream (Clotrimazole 1% / Betamethasone dipropionate 0.05%), include paraesthesia, maculopapular rash, oedema and secondary infection. Common side effects reported for others commercially available combinations, Canesten Hydrocortisone (Clotrimazole 1% / Hydrocortisone 1%) and Lotriderm (Clotrimazole 1% / Betamethasone dipropionate 0.05%) are local mild burning, irritation and hypersensitivity reactions. Moreover, such combination products sometimes fail to provide the fast relief of the inflammatory symptoms which is normally desired for the treatment of a fungal infection.

It is known that fungal infections of the skin can be later colonized by bacteria. These bacteria contribute to the perpetuation of the injury and of the clinical symptoms.

### Description of the invention

The problem to be solved by the present invention is to provide a pharmaceutical composition useful to treat dermal diseases, caused by fungi and yeasts with inflammation and/or associated with a bacterial infection, which provides a fast relief of the symptomatology and reduces side effects. The solution is based on the fact that the applicants have found that pharmaceutical compositions, which comprise
a) Sertaconazole, or its pharmaceutically acceptable salts, solvates, isomers or mixtures thereof, and
b) Hydrocortisone, or pharmaceutically acceptable esters thereof or mixtures thereof, or an antibacterial agent, or a mixture thereof,
have an improved pharmacological profile as compared to other compositions of Sertaconazole alone and/or other antifungals in combination with anti-inflammatory agents.

Accordingly, a first aspect of the invention relates to a pharmaceutical composition comprising:
a) a therapeutically effective amount of Sertaconazole, or a pharmaceutically acceptable salt, solvate, isomer thereof or mixtures thereof; and
b) a therapeutically effective amount of at least one compound selected from the group consisting of Hydrocortisone, a pharmaceutically acceptable ester of Hydrocortisone, an antibacterial agent or mixtures thereof; optionally in combination with at least one pharmaceutically acceptable excipient or carrier.

Accordingly, the present invention relates to a pharmaceutical composition as defined above to be used in the treatment of fungal infections, caused by fungi and yeasts with inflammation and/or associated with a bacterial infection.

According to a second aspect, the present invention relates to the use of a pharmaceutical composition as defined above, for the manufacture of a medicament for the treatment of fungal infections, caused by fungi and yeasts with inflammation and/or associated with a bacterial infection, in a human or animal living body, by administering an effective amount of the composition according to the invention. Preferably, the pharmaceutical composition of the invention is used in a human.

This second aspect may alternatively be formulated as a method for treatment of the diseases mentioned above in a human or animal living body, comprising administering to a human or animal living body in need thereof an effective amount of a pharmaceutical composition as described herein. Preferably, the method of treatment comprises administration of the pharmaceutical composition as described herein in a human.

Fungal diseases refer to fungal infections, including yeast infections, of keratinized and non-keratinized epithelial tissues, for example skin, nails, mucosa and the like. Thus, the compositions of the invention are useful to treat fungal diseases with itching and/or inflammation, such as tinea pedis, tinea capitis, tinea corporis, tinea versicolor, and tinea cruris, nail fungal diseases (onychomycosis caused by dermatophyte and yeast infection). Other indications are for yeast diseases, such as candidiasis and itertriginous dermatitis, in which the presence of pathogenic yeast organisms, causes skin disease with resultant inflammation and itching. The compositions may also be useful to treat fungal diseases associated with a bacterial infection.

For the purposes of the present invention, the Sertaconazole compound may be Sertaconazole, its pharmaceutically acceptable salts, solvates and isomers. Particularly preferred is R-(-)-Sertaconazole and its pharmaceutically acceptable salts and solvates. Pharmaceutically acceptable solvates may be hydrates or comprising other solvents of crystallization such as alcohols. Representative pharmaceutically acceptable salts include hydrochloride, hydrobromide, sulphate, bisulphate, nitrate, phosphate, perchlorate, borate, acetate, tartrate, maleate, citrate, succinate, palmoate, methanesulfonate, benzoate, salicylate, and the like.

Preferably, the Sertaconazole compound is selected from Sertaconazole base, Sertaconazole mononitrate, R-Sertaconazole base and R-Sertaconazole mononitrate.

For the purposes of the present invention, Hydrocortisone refers to Hydrocortisone base or their pharmaceutically acceptable esters. Hydrocortisone and its esters could be classified according to their potency, e.g. of low-potency Hydrocortisone are Hydrocortisone base and Hydrocortisone acetate, e.g. of mid-potency Hydrocortisone are Hydrocortisone butyrate, Hydrocortisone propionate and Hydrocortisone valerate.

In a preferred embodiment, the Hydrocortisone is a low potency Hydrocortisone. In another preferred embodiment, the Hydrocortisone is a mid-potency Hydrocortisone.

In a more preferred embodiment, esters of Hydrocortisone are Hydrocortisone aceponate, Hydrocortisone acetate, Hydrocortisone buteprate, Hydrocortisone butylacetate, Hydrocortisone butyrate, Hydrocortisone cypionate, Hydrocortisone propionate and Hydrocortisone valerate.

Particularly preferred are Hydrocortisone base, Hydrocortisone acetate, Hydrocortisone butyrate and Hydrocortisone valerate.

The antibiotics utilized in the present invention may be generally described as being selected from the group consisting of tetracyclines (e.g. Tetracycline and Doxycycline), aminoglycosides (e.g. Amikacin, Gentamicin, Kanamycin, Neomycin, Netilmicin, Paromomycin, Streptomycin and Tobramycin), penicillins (e.g. Penicillin and Amoxicillin), cephalosporins (e.g. Cefaclor, Cefadroxil, Cefazolin, Cefixime, Cefoxitin, Cefprozil, Ceftazidime, Cefuroxime and Cephalexin), quinolones (e.g. Ciprofloxacin, Moxifloxacin, Ofloxacin, Gatifloxacin, Levofloxacin, Norfloxacin, Enterofloxacin, GF-001001-00 and Trovafloxacin), carbapenems (e.g. Imipenem and Carbapenem) and erythromycins (e g. Erythromycin, Azithromycin and Clarithromycin)

In a preferred embodiment, the antibacterial agent is a quinolone. In a more preferred embodiment, the antibacterial agent is 1-cyclopropyl-8-methyl-7-[5-methyl-6-(methylamino)-3-pyridinyl]-4-oxo-1,4-dihydro-3-quinolinecarboxylic acid (GF-001001-00).

The amount of the active principle ingredient utilized in the compositions of the present invention will depend on the purpose of the use, e.g., the treatment of an active infection, the prophylactic treatment of tissues to prevent an active infection from developing, or the sterilization of tissues in conjunction with a medical procedure, such as a surgical procedure. The amounts utilized will also depend on the particular tissues being treated. For example, lower concentrations will typically be utilized to treat especially sensitive tissue, while somewhat higher concentrations may be utilized to treat less sensitive tissues.

The compositions of the invention contain from about 0.1 % to about 10 % by weight of the composition of Sertaconazole, or a pharmaceutically acceptable salts, solvates, isomers or mixtures thereof. Preferably the amount of Sertaconazole or a pharmaceutically acceptable salts, solvates, isomers or mixtures thereof in the composition is from about 0.2 % to about 8 % by weight of the composition. More preferably, from about 0.2 % to about 7 % by weight of the composition. In another preferred embodiment of the invention, R-Sertaconazole base and R-Sertaconazole mononitrate are in an amount from about 0.3 % to about 5 % by weight of the composition. In another preferred embodiment, Sertaconazole base and Sertaconazole mononitrate are in an amount from about 0.5 % to about 5% by weight of the composition.

The compositions of the invention contain from about 0.05 % to about 5 % by weight of the composition of Hydrocortisone or a pharmaceutically acceptable ester or mixtures thereof. Preferably, from about 0.1 % to about 2 % by weight of the composition.

The compositions of the invention contain from about 0.5 % to about 10.0 % by weight of an antibacterial agent.

An embodiment of the first aspect, the invention relates to pharmaceutical compositions comprising an effective amount of Sertaconazole, their pharmaceutically acceptable salts, solvates and isomers and at least one Hydrocortisone or their pharmaceutically acceptable esters, resulting in a synergistic effect for the treatment of fungal diseases caused by fungi or yeasts with itching and/or inflammation.

In another embodiment, the invention relates to pharmaceutical compositions comprising an effective amount of Sertaconazole, its pharmaceutically acceptable salts, solvates and isomers and at least one antibacterial agent. In a more preferred embodiment, the antibacterial agent is a quinolone. In an even more preferred embodiment, the antibacterial agent is 1-cyclopropyl-8-methyl-7-[5-methyl-6-(methylamino)-3-pyridinyl]-4-oxo-1,4-dihydro-3-quinolinecarboxylic acid (GF-001001-00).

These compositions, which contain an antibacterial agent, have a synergistic effect for the treatment of bacterially infected fungal diseases, such as fungal infections caused by Gram-positive and Gram-negative organisms including drug-resistance bacteria.

In another embodiment, the invention relates to pharmaceutical compositions comprising an effective amount of Sertaconazole, or its pharmaceutically acceptable salts, solvates and isomers thereof and at least one Hydrocortisone or their pharmaceutically acceptable esters and at least one antibacterial agent. These compositions are endowed with a synergistic effect for the treatment of bacterially infected fungal diseases complicated by itching and/or inflammation.

According to a preferred embodiment, the invention relates to a pharmaceutical composition comprising a Sertaconazole compound selected from Sertaconazole base, Sertaconazole mononitrate, R-Sertaconazole base and R-Sertaconazole mononitrate, and a Hydrocortisone selected from the group consisting of Hydrocortisone base, Hydrocortisone valerate, Hydrocortisone acetate and Hydrocortisone butyrate, in combination with pharmaceutically acceptable carriers.

According to a preferred embodiment, the invention relates to a pharmaceutical composition comprising a Sertaconazole compound selected from Sertaconazole base, Sertaconazole mononitrate, R-Sertaconazole base and R-Sertaconazole mononitrate, and an antibiotic agent, in combination with pharmaceutically acceptable carriers. Particularly preferred are those compositions wherein the antibiotic agent is a quinolone. Most preferred are those wherein the antibiotic agent is 1-cyclopropyl-8-methyl-7-[5-methyl-6-(methylamino)-3-pyridinyl]-4-oxo-1,4-dihydro-3-quinolinecarboxylic acid (GF-001001-00).

In another preferred embodiment, the invention relates to a pharmaceutical composition comprising a Sertaconazole compound selected from Sertaconazole base, Sertaconazole mononitrate, R-Sertaconazole base and R-Sertaconazole mononitrate, and a Hydrocortisone selected from the group consisting of Hydrocortisone base, Hydrocortisone valerate, Hydrocortisone acetate and Hydrocortisone butyrate, and an antibiotic agent, in combination with pharmaceutically acceptable carriers. Particularly preferred are those compositions wherein the antibiotic agent is a quinolone. Most preferred are those wherein the antibiotic agent is 1-cyclopropyl-8-methyl-7-[5-methyl-6-(methylamino)-3-pyridinyl]-4-oxo-1,4-dihydro-3-quinolinecarboxylic acid (GF-001001-00).

The route of administration of the compositions may be ocular, nasal, otic, rectal, vaginal, intradermal, intratumoral, intralesional, intravascular, topical, transdermal, local or regional. In a preferred embodiment, the invention relates to topical compositions. In an embodiment of the invention, the composition is designed for topical administration.

The compositions of the present invention may be formulated in any dosage form such as creams, foams, pastes, ointments, emulsions, milks, pomades, powders, solutions, gels, sprays, shampoos, lotions, suspensions, microspheres, microcapsules, nanospheres, nanoparticles, lipidic vesicles, liposomes, polymeric vesicles, patches or biological inserts.

According to a preferred embodiment, the invention relates to anhydrous compositions in the form of anhydrous microemulsion or anhydrous cream.

The formulation described herein optionally contains one or more other pharmaceutically active agents. Useful agents include any agents commonly used in dermatological formulations, which may include, but are not limited to, antibacterial agents such as those cited above, and other antiiflammatory agents.

In addition to the active pharmaceutical ingredients (APIs) mentioned above, the compositions of the invention will also include pharmaceutically acceptable excipients known in the art for pharmaceutical compounding such as for example, solvents, buffering agents, stabilizing agents, penetration enhancers, humectants and/or moisturizers, preservatives, opacifiers, fragrances, colour additives, emulsifying agents, bases, emollients, stiffening agents, solubilizing agents and the like.

In an embodiment of the process of the present invention, the present compositions may be prepared using conventional techniques, for example, by formation of solutions, gels, suspensions, etc., using well known and conventional techniques. Compositions of the present invention can also be prepared by processes known in the art, including by simple admixture, with agitation as appropriate, of the ingredients.

Generally a composition for topical use is applied once or more times per day on the area to be treated. The number of times per day that the composition is applied depends on the severity of the condition and the advice of the physician. The pharmacological profile of the compositions of the invention may be evaluated in patients suffering from fungal and yeast infections with inflammation and/or associated with a bacterial infection.

Additional objects, advantages and features of the invention will become apparent to those skilled in the art upon examination of the description or may be learned by practice of the invention. The following examples are provided by way of illustration and are not intended to be limiting to the present invention.

### EXAMPLES

### Example 1. 1 % Sertaconazole mononitrate + 0.1% Hydrocortisone acetate anhydrous microemulsion

This example illustrates the present invention in the form of an anhydrous microemulsion of Sertaconazole mononitrate and Hydrocortisone acetate. The pharmaceutical composition of this example is given below:

| | |
|---|---|
| Sertaconazole mononitrate | 1.00 g |
| Hydrocortisone acetate | 0.10 g |
| Labrasol | 52.50 g |
| Plurol oleic | 13.40 g |
| Myritol 318 | 15.00 g |
| Propylene glycol | 18.00 g |

Labrasol, Plurol oleic, Myritol 318 and Propylene glycol were mixed and heated to 35-40 °C. Sertaconazole mononitrate and Hydrocortisone acetate were added under magnetic stirring until total dissolution, after that the microemulsion was cooled and stored.

Based on the method described in Example 1, the following compositions may be prepared.

### Example 2. 2% Sertaconazole nitrate + 0.3% Hydrocortisone valerate anhydrous cream

This example illustrates the present invention in the form of an anhydrous cream of Sertaconazole mononitrate and Hydrocortisone valerate. The pharmaceutical composition of this example is given below:

| | |
|---|---|
| Sertaconazole nitrate | 2.00 g |
| Hydrocortisone valerate | 0.30 g |
| Eumulgin B3 | 20 g |
| Isopropyl palmitate | 10 g |
| Liquid Paraffin q.s. | 100 g |

### Example 3. 1% R-Sertaconazole + 0.5 Hydrocortisone valerate anhydrous cream

This example illustrates the present invention in the form of an anhydrous cream of R-Sertaconazole and Hydrocortisone valerate. The pharmaceutical composition of this example is given below:

| | |
|---|---|
| R-Sertaconazole | 1.00 g |
| Hydrocortisone valerate | 0.50 g |
| Glyceril monoestearate | 6 g |
| Miglyol 812 | 20 g |
| Miglyol 840 | 10 g |
| Liquid paraffin | 14 g |
| Softisan 378 q.s. | 100 g |

### Example 4. 2% Sertaconazole nitrate + 0.5% Hydrocortisone acetate + 1% GF-001001-00 anhydrous cream

This example illustrates the present invention in the form of an anhydrous cream of Sertaconazole mononitrate, Hydrocortisone acetate and GF-001001-00. The pharmaceutical composition of this example is given below:

| | |
|---|---|
| Sertaconazole nitrate | 2.00 g |
| Hydrocortisone acetate | 0.50 g |
| GF-001001-00 | 1.00 g |
| Miglyol 812 | 20 g |
| White soft paraffin | 20 g |
| Liquid paraffin | 10 g |
| Softisan 378 q.s. | 100 g |

### Example 5. 1% Sertaconazole + 0.5% Hydrocortisone acetate + 1% GF-001001-00 anhydrous cream

This example illustrates the present invention in the form of an anhydrous cream of R-Sertaconazole, Hydrocortisone acetate and GF-001001-00. The pharmaceutical composition of this example is given below:

| | |
|---|---|
| R-Sertaconazole | 1.00 g |
| Hydrocortisone acetate | 0.50 g |
| GF-001001-00 | 1.00 g |
| Cetyl alcohol | 3 g |
| Lanoline | 2 g |
| Arlatone T | 20 g |
| White soft paraffin q.s. | 100 g |

### Example 6. 2% Sertaconazole + 0.3% Hydrocortisone acetate + 1% GF-001001-00 o/w emulsion

This example illustrates the present invention in the form of an o/w emulsion of Sertaconazole mononitrate, Hydrocortisone acetate and GF-001001-00. The pharmaceutical composition of this example is given below:

| | |
|---|---|
| Sertaconazole mononitrate | 2.00 g |
| Hydrocortisone acetate | 0.30 g |
| GF-001001-00 | 1.00 g |
| Tefose 1500 | 15.00 g |
| Stearic acid | 2.00 g |
| Mineral oil | 6.00 g |
| Methyl paraben sodium salt | 0.05 g |
| Sorbic acid | 0.05 g |
| Purified water | 69.6 g |
| Labrafil M2130 CS | 5.00 g |

### Example 7. 1% Sertaconazole + 0.3% Hydrocortisone acetate + 1% GF-001001-00 o/w emulsion

This example illustrates the present invention in the form of an o/w emulsion of R-Sertaconazole, Hydrocortisone acetate and GF-001001-00. The pharmaceutical composition of this example is given below:

| | |
|---|---|
| R-Sertaconazole mononitrate | 1.00 g |
| Hydrocortisone acetate | 0.30 g |
| GF-001001-00 | 1.00 g |
| Tefose 1500 | 15.00 g |
| Stearic acid | 2.00 g |
| Mineral oil | 6.00 g |
| Methyl paraben sodium salt | 0.05 g |
| Sorbic acid | 0.05 g |
| Labrafil M2130 CS | 5.00 g |
| Purified water | 69.6 g |

### Example 8. 2% Sertaconazole + 1% GF-001001-00 anhydrous cream

This example illustrates the present invention in the form of an anhydrous cream of Sertaconazole mononitrate, and GF-001001-00. The pharmaceutical composition of this example is given below:

| | |
|---|---|
| Sertaconazole mononitrate | 2.00 g |
| GF-001001-00 | 1.00 g |
| Sedefos 75 | 20.00 g |
| Compritol 888 ATO | 3.00 g |
| White paraffin | 5.00 g |
| Octyldodecyl myristate | 5.00 g |
| PEG 300 | 65.00 g |

### Example 9. 1% R-Sertaconazole + 1% GF-001001-00

This example illustrates the present invention in the form of an anhydrous cream of R-Sertaconazole mononitrate, and GF-001001-00. The pharmaceutical composition of this example is given below:

| | |
|---|---|
| R-Sertaconazole mononitrate | 1.00 g |
| GF-001001-00 | 1.00 g |
| Sedefos 75 | 20.00 g |
| Compritol 888 ATO | 3.00 g |
| White paraffin | 5.00 g |
| Octyldodecyl myristate | 5.00 g |
| PEG 300 | 65.00 g |

### Example 10.

The principal objective of this study was to determine that the pharmaceutical compositions as described herein have improved the pharmacological profile with a fast relief of the symptomatology and reduced side effects.

The compositions as described in Examples 1 to 10 were tested against fungal and yeast infections with inflammation and/or associated with a bacterial infection.

The results have shown that all the compositions presented an improved pharmaceutical profile, with a fast relief of the symptomatology, reduction of inflammation, prevention of bacterial infection and decrease of side effects.

## Claims

1. A pharmaceutical composition comprising:
a) a therapeutically effective amount of Sertaconazole, or a pharmaceutically acceptable salt, solvate, isomer thereof or mixtures thereof; and
b) a therapeutically effective amount of at least one compound selected from the group consisting of Hydrocortisone, a pharmaceutically acceptable ester of Hydrocortisone, an antibacterial agent or mixtures thereof; optionally in combination with at least one pharmaceutically acceptable excipient or carrier.

2. The pharmaceutical composition according to claim 1 wherein the Sertaconazole is selected from the group consisting of Sertaconazole base, R-Sertaconazole base, Sertaconazole mononitrate and R-Sertaconazole mononitrate.

3. The pharmaceutical composition according to any of claims 1 to 2 wherein the Hydrocortisone is selected from the group consisting of Hydrocortisone base, Hydrocortisone aceponate, Hydrocortisone acetate, Hydrocortisone buteprate, Hydrocortisone butylacetate, Hydrocortisone butyrate, Hydrocortisone cypionate, Hydrocortisone propionate and Hydrocortisone valerate.

4. The pharmaceutical composition according to any of claims 1 to 3 wherein the Hydrocortisone is selected from the group consisting of Hydrocortisone base, Hydrocortisone acetate, Hydrocortisone butyrate and Hydrocortisone valerate.

5. The pharmaceutical composition according to any of claims 1 to 4 wherein the therapeutically effective amount of Sertaconazole, or a pharmaceutically acceptable salt, solvate, isomer or mixtures thereof, ranges from about 0.1 % to about 10 % by weight of the composition.

6. The pharmaceutical composition according to any of claims 1 to 5 wherein the therapeutically acceptable amount of Sertaconazole, or a pharmaceutically acceptable salt, solvate, isomer or mixtures thereof ranges from about 0.2 % to about 8 % by weight of the composition.

7. The pharmaceutical composition according to any of claims 1 to 6 wherein R-Sertaconazole or R-Sertaconazole mononitrate is present in an amount ranging from about 0.3 % to about 5% by weight of the composition.

8. The pharmaceutical composition according to any of claims 1 to 6 wherein Sertaconazole base or Sertaconazole mononitrate is present in an amount ranging from about 0.5 % to about 5% by weight of the composition.

9. The pharmaceutical composition according to any of claims 1 to 8 wherein the therapeutically acceptable amount of Hydrocortisone or a pharmaceutically acceptable ester or mixtures thereof ranges from about 0.05 % to about 5 % by weight of the composition.

10. The pharmaceutical composition according to any of claims 1 to 9 wherein the therapeutically acceptable amount of Hydrocortisone or a pharmaceutically acceptable ester or mixtures thereof ranges from about 0.1 % to about 2 % by weight of the composition.

11. The pharmaceutical composition according to any of claims 1 to 10 wherein the antibacterial agent is a quinolone.

12. The pharmaceutical composition according to any of claims 1 to 11 wherein the antibacterial agent is 1-cyclopropyl-8-methyl-7-[5-methyl-6-(methylamino)-3-pyridinyl]-4-oxo-1,4-dihydro-3-quinolinecarboxylic acid.

13. The pharmaceutical composition according to any of claims 1 to 12 wherein the therapeutically acceptable amount of the antibacterial agent ranges from about 0.5 % to about 10 % by weight of the composition.

14. The composition according to any of claims 1 to 13 wherein such composition is designed for topical administration.

15. The composition according to claim 14 wherein the composition is formulated as creams, foams, pastes, ointments, emulsions, milks, pomades, powders, solutions, gels, sprays, shampoos, lotions, suspensions, microspheres, microcapsules, nanospheres, nanoparticules, lipidic vesicles, liposomes, polymeric vesicles, patches or biological inserts.

16. The composition according to any of claims 14 to 15, wherein the composition is formulated as in an anhydrous microemulsion or an anhydrous cream.

17. The use of the pharmaceutical composition according to any of claims 1 to 16, for the manufacture of a medicament for the treatment of fungal infections, caused by fungi and yeasts with inflammation and/or associated with a bacterial infection, in a human or animal living body.

18. The use according to claim 17 wherein the fungal infection is selected from the group consisting of tinea pedis, tinea capitis, tinea corporis, tinea versicolor, and tinea cruris, nail fungal diseases, candidiasis and itertriginous dermatitis, and fungal diseases associated with a bacterial infection.
